# EUROPEAN PATENT SPECIFICATION

(11) **EP 1 097 212 B1**
(45) Date of publication and mention of the grant of the patent: **24.12.2008**
(21) Application number: 99941953.4
(22) Date of filing: 09.07.1999
(51) Int. Cl.: C12N 15/31, C07K 14/32, A61K 39/07, C12N 7/01, C12N 1/21

(54) **ANTHRAX VACCINE**
ANTHRAX-IMPFSTOFF
VACCIN DU CHARBON BACTERIDIEN

(30) Priority: 10.07.1998 US 92416 P
(43) Date of publication of application: 09.05.2001
(73) Proprietor: U.S. Medical Research Institute of Infectious Diseases, Frederick, MD 21702 (US)
(72) Inventor: LEE, John, S., Hagerstown, MD 21740 (US); PUSHKO, Peter, Frederick, MD 21701 (US); PARKER, Michael, D., Frederick, MD 21701 (US); SMITH, Jonathan, F., Sabillasville, MD 21780 (US); WELKOS, Susan, L., Frederick, MD 21701 (US)
(74) Representative: UEXKÜLL & STOLBERG
(86) International application number: PCT/US1999/015568
(87) International publication number: WO 2000/002522

(56) References cited:
- WO-A-95/07994
- WO-A-96/17067
- WO-A-96/37616
- WO-A-98/08952
- L.C. IACONO-CONNORS ET AL.,: "PROTECTION AGAINST ANTHRAX WITH RECOMBINANT VIRUS-EXPRESSED PROTECTIVE ANTIGEN IN EXPERIMENTAL ANIMALS" INFECTION AND IMMUNITY, vol. 59, no. 6, June 1991 (1991-06), pages 1961-1965, XP000916157
- PUSHKO P ET AL: "Replicon-helper systems from attenuated Venezuelan Equine Encephalitis virus: Expression of heterologous genes in vitro and immunization against heterologous pathogens in vivo" VIROLOGY,US,ACADEMIC PRESS,ORLANDO, vol. 239, no. 2, 22 December 1997 (1997-12-22), pages 389-401-401, XP002123951 ISSN: 0042-6822
- SINGH Y ET AL: "A DELETED VARIANT OF BACILLUS ANTHRACIS PROTECTIVE ANTIGEN IS NON- TOXIC AND BLOCKS ANTHRAX TOXIN ACTION IN VIVO" JOURNAL OF BIOLOGICAL CHEMISTRY,US,AMERICAN SOCIETY OF BIOLOGICAL CHEMISTS, BALTIMORE, MD, vol. 264, no. 32, 15 November 1989 (1989-11-15), pages 19103-19107, XP000650160 ISSN: 0021-9258
- IVINS B E ET AL: "CLONING AND EXPRESSION OF THE BACILLUS ANTHRACIS PROTECTIVE ANTIGENGENE IN BACILLUS SUBTILIS" INFECTION AND IMMUNITY,US,AMERICAN SOCIETY FOR MICROBIOLOGY. WASHINGTON, vol. 54, no. 2, 1 November 1986 (1986-11-01), pages 537-542, XP000650052 ISSN: 0019-9567
- DAVIS N L; WILLIS L V; SMITH J F; JOHNSTON R E: "IN-VITRO SYNTHESIS OF INFECTIOUS VENEZUELAN EQUINE ENCEPHALITIS VIRUS RNA FROM A COMPLEMENTARY DNA CLONE ANALYSIS OF A VIABLE DELETION MUTANT" VIROLOGY, vol. 171, July 1989 (1989-07), - July 1989 (1989-07) pages 189-204,

## Description

### FIELD OF THE INVENTION

This invention relates to vaccines for bacterial toxins from *Bacillus anthracis*.

### INTRODUCTION

Anthrax is a disease cause by the sporulating bacteria *Bacillus anthracis.* Humans working with animal products are at risk from contracting anthrax. Areas such as Iran, Turkey, Iraq, Pakistan, and sub-Saharan Africa are hyperendemic for anthrax, although the organism can be found in most areas of the world. Anthrax manifests disease in three different ways. Inhalation, gastrointestinal, and cutaneous anthrax result from inhaling spores, ingesting spores in contaminated meat, or contacting spores in an open wound, respectively. Untreated inhalation or gastrointestinal anthrax has a case fatality rate of essentially 100 percent while cutaneous anthrax has a case fatality rate of up to 25 percent. Previous research has shown that the protective antigen (PA) produced by *B. anthracis* can protect mice from anthrax. Even though the Anthrax vaccine is FDA licensed, reactogenicity is mild to moderate.

Therefore, there is a need for an efficacious vaccine for anthrax useful for protecting humans.

### SUMMARY OF THE INVENTION

The present invention satisfies the need discussed above. The present invention relates to a method and composition for use in inducing an immune response which is protective against infection with anthrax.

In this vaccine strategy, a gene coding for a protein of interest is cloned into a VEE virus vector in place of the VEE virus structural genes; the result is a self-replicating RNA molecule, a replicon, that encodes its own replicase and transcriptase functions, and in addition makes abundant quantities of mRNA encoding the foreign protein. When replicon RNA is transfected into eukaryotic cells along with two helper RNAs that express the VEE structural proteins (glycoproteins and nucleocapsid), the replicon RNA is packaged into VEE virus-like particles by the VEE virus structural proteins, which are provided in trans. Since the helper RNAs lack packaging signals neccessary for further propagation, the resulting VEE replicon particles (VRPs) which are produced are infectious for one cycle but are defective thereafter. Upon infection of an individual cell with a VRP, an abortive infection occurs in which the infected cell produces the protein of interest in abundance, is ultimately killed by the infection, but does not produce any viral progeny (Pushko et al., 1997, Virology 239, 389-401).

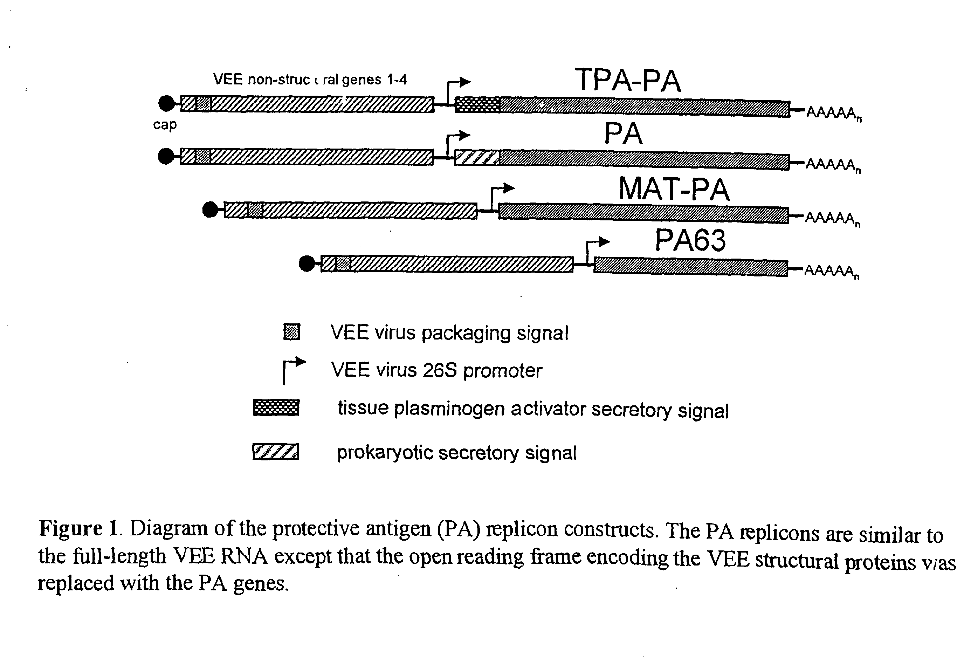

The PA gene which contains a prokaryotic secretory signal and the entire 83 kDa coding sequence (Welkos et al., 1988, Gene 69, 287-300), was inserted into the VEE replicon vaccine vector (Figure 1) and have demonstrated high level expression of this bacterial protein in eukaryotic cells in culture. Mice, either the C57BL/6 strain or the A/J strain, inoculated with VRP containing the PA-replicon produced high specific antibody titers and were protected from developing anthrax when challenged subcutaneously with *B. anthracis.*

Therefore, it is one object of the present invention to provide a VEE virus replicon vector comprising a VEE virus replicon and DNA fragments encoding the PA protein from *B. anthracis.*

It is another object of the present invention to provide a self replicating RNA comprising the VEE virus replicon and any of the *B. anthracis* fragments described above.

It is another object of the present invention to provide infectious VEE virus replicon particles produced from the VEE virus replicon RNA described above.

It is further an object of the invention to provide an immunological composition for the protection of mammals against *B. anthracis* infection comprising VEE virus replicon particles containing any of the *B. anthracis* fragments described above or a combination of different VEE virus replicons each having a different *B. anthracis* fragment.

### BRIEF DESCRIPTION OF THE DRAWINGS

These and other features, aspects, and advantages of the present invention will become better understood with reference to the following description and appended claims, and accompanying drawings where:
**Figure 1**. Diagram of the protective antigen (PA) replicon constructs. The PA replicons are similar to the full-length VEE RNA except that the open reading frame encoding the VEE structural proteins was replaced with the PA genes.
**Figure 2****.** Western blot of BHK cell lysates showing expression of anthrax protective antigen from recombinant VEE replicons. a) uninfected cell lysate; b) recombinant expression product from *B. anthracis;* c) infected cell lysate.

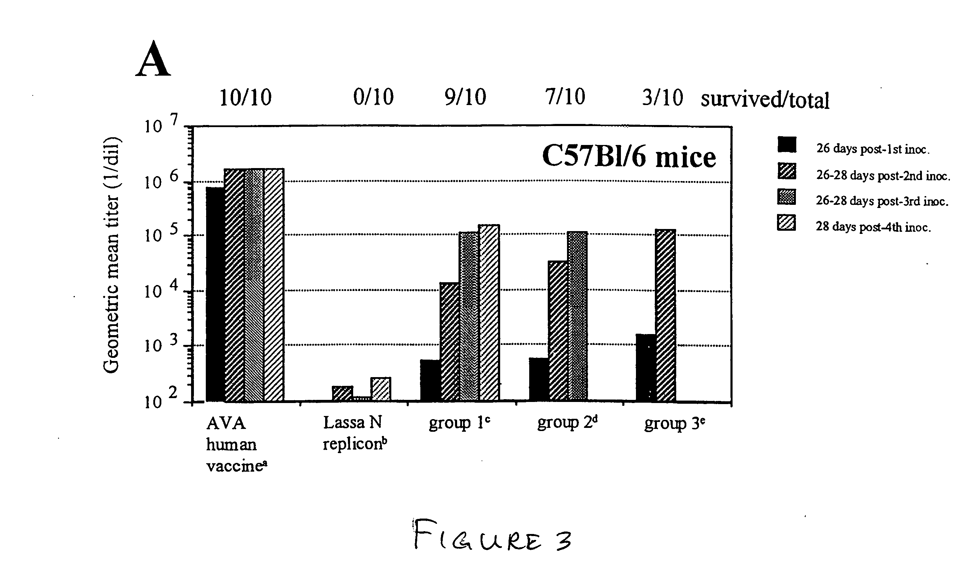

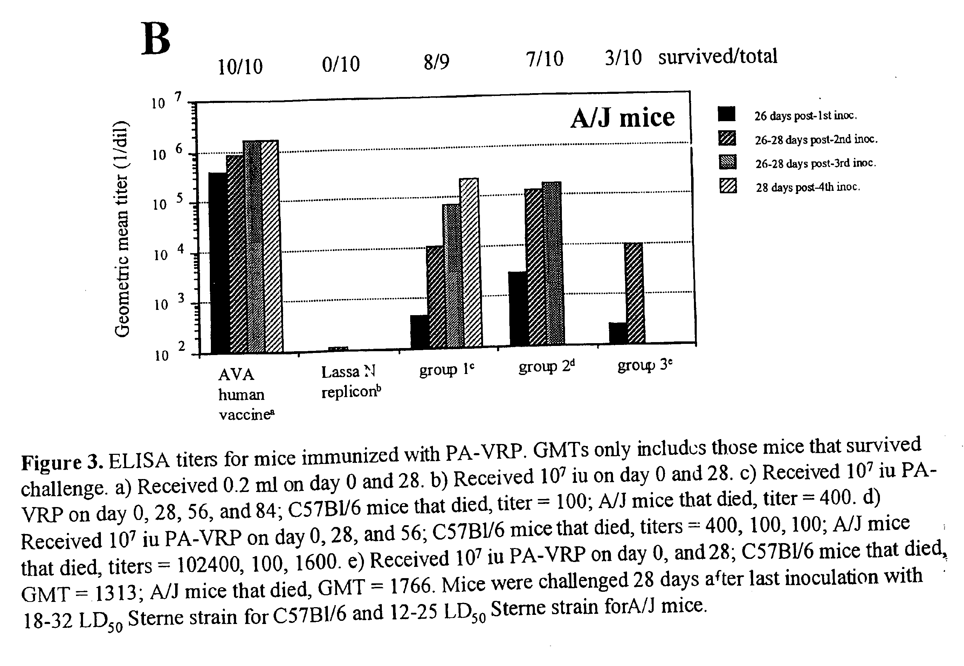

**Figure 3A** **and** **3B****.** ELISA titers for mice (A, C57B1/6 mice; B, A/J mice) immunized with PA-VRP. GMTs only includes those mice that survived challenge. a) Received 0.2 ml on day 0 and 28. b) Received 10⁷ iu on day 0 and 28. c) Received 10⁷ iu PA-VRP on day 0, 28, 56, and 84; C57B1/6 mice that died, titer = 100; A/J mice that died, titer = 400. d) Received 10⁷ iu PA-VRP on day 0, 28, and 56; C57B1/6 mice that died, titers = 400, 100, 100; A/J mice that died, titers = 102400, 100, 1600. e) Received 10⁷ iu PA-VRP on day 0, and 28; C57B1/6 mice that died, GMT = 1313; A/J mice that died, GMT = 1766. Mice were challenged 28 days after last inoculation with 18-32 LD₅₀ Sterne strain for C57B1/6 and 12-25 LD₅₀ Sterne strain forA/J mice.

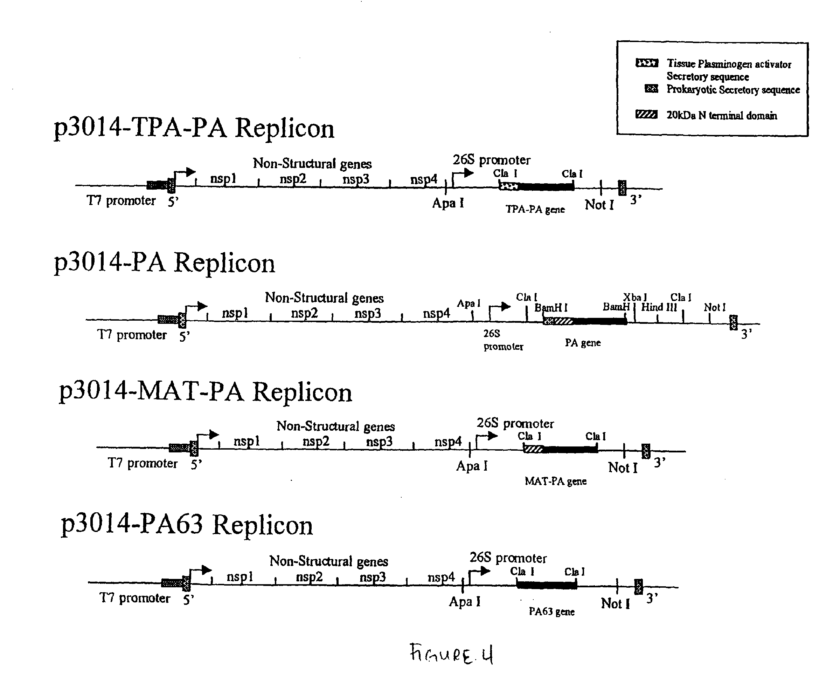

**Figure 4****.** Schematic representation of constructs containig PA.

### DETAILED DESCRIPTION

In the description that follows, a number of terms used in recombinant DNA, virology and immunology are extensively utilized. In order to provide a clearer and consistent understanding of the specification and claims, including the scope to be given such terms, the following definitions are provided.

**Replicon.** A replicon is equivalent to a full length virus from which all of the viral structural proteins have been deleted. A multiple cloning site can be cloned into the site previously occupied by the structural protein genes. Virtually any heterologous gene may be cloned into this cloning site. Transcription of the RNA from the replicon yields an RNA capable of initiating infection of the cell identically to that seen with the full-length infectious virus clone. However, in lieu of the viral structural proteins, the heterologous antigen is expressed. This system does not yield any progeny virus particles because there are no viral structural proteins available to package the RNA into particles.

Particles which appear structurally identical to virus particles can be produced by supplying structural proteisn for packaging of the replicon RNA *in trans.* This is typically done with two helpers also called defective helper RNAs. One helper consists of a full length infectious clone from which the nonstructural protein genes and the glycoprotein genes are deleted. The helper retains only the terminal nucleotide sequences, the promoter for subgenomic mRNA transcription and the sequences for the viral nucleocapsid protein. The second helper is identical to the first except that the nucleocapsid gene is deleted and only the glycoprotein genes are retained. The helper RNA's are transcribed *in vitro* and co-transfected with replicon RNA. Because the replicon RNA retains the sequences for packaging by the nucleocapsid protein, and because the helpers lack these sequences, only the replicon RNA is packaged by the viral structural proteins and released from the cell. The particles can then by inoculated into animals similar to parent virus. The replicon particles will initiate only a single round of replication because the helpers are absent, they produce no progeny virus particles, and express only the viral nostructural proteins and the product of the heterologous gene cloned in place to the structural proteins.

The VEE virus replicon is a genetically reorganized version of the VEE virus genome in which the structural proteins genes are replaced with a gene from an immunogen of interest, in this invention, the *B. anthracis* proteins. The result is a self replicating RNA (replicon) that can be packaged into infectious particles using defective helper RNAs that encode the glycoprotein and capsid proteins of the VEE virus.

**Subject.** Includes both human, animal, e.g., horse, cattle, donkey, monkey, pig, dog, guinea pig, mouse, hamster, avian e.g., chicken, pheasant or turkey, fish and other marine animals, and insects such as mosquito.

In one embodiment, the present invention relates to a recombinant DNA molecule that includes a VEE replicon and a DNA sequence encoding a non-toxic protective antigen (PA) from *B*. *anthracis*, MAT-PA protein having the amino acid sequence of SEQ ID NO : 7. The sequence for PA has been determined and has been deposited in GenBank at accession no. M22589. The PA sequence encodes a prokaryotic secretory signal in addition to the entire sequence encoding the 83 Kd PA (SEQ ID NO:1). Other nucleic acid sequences related to PA include sequences wherein the secretory signal has been-removed (MAT-PA)(SEQ ID NO:2), or replaced with other secretory signals known to people in the art such as the tissue plasminogen activator (TPA) secretory signal resulting in a DNA fragment encoding TPA-PA (SEQ ID NO:3). Further nucleic acid sequences related to PA are sequences encoding the active form of PA, a 63 kDa protein, termed PA63 (SEQ ID NO:4). In addition, isolated nucleic acid molecules related to PA include DNA molecules which comprise a sequence substantilly differenct from those described above but which; due to the degeneracy of the genetic code, still encode the *B*. *anthracis* proteins described and specified in SEQ ID NO:5 (TPA-PA), SEQ ID NO:6 (PA), and SEQ ID NO:8 (PA63). Of course, the genetic code and species-specific codon preferences are well known in the art. Thus, it would be routine for one skilled in the art to generate the degenerate variants described above, for instance, to optimize codon expression for a particular host (e.g., change codons in the human mRNA to those preferred by a bacterial host such as *E.coli*).

Nucleic acid molecules of the present invention may be in the form of RNA, or in the form of DNA, including, for instance, cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the antisense strand.

By "isolated" nucleic acid molecule(s) is intended a nucleic acid molecule, DNA or RNA, which has been removed from its native environment. For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically.

Variants of the nucleic acid molecules described herein encode portions, analogs or derivatives of the *B. anthracis* polypeptides described above. Variants may occur naturally, such as a natural allelic variant. By an "allelic variant" is intended one of several alternate forms of a gene occupying a given locus of a chromosome of an organism. Non-naturally occuring variants may be produced by known mutagenesis techniques. Such variants include those produced by nucleotide substitution, deletion, or addition of one or more nucleotides in the coding or noncoding regions or both. Alterations in the coding regions may produce conservative or nonconservative amino acid substitutions, deletions, or additions. Especially useful among these are silent substitutions, additions, and deletions which do not alter the properties and activities of the *B. anthracis* polypeptides disclosed herein or portions thereof. Also useful in this regard are conservative substitutions.

In another embodiment, the present invention relates to a recombinant DNA construct that includes a VEE virus replicon vector and a DNA sequence encoding B. anthracis MAT-PA protein having the amino acid sequence of SEQ NO:7.

The cloned gene may optionally be placed under the control of (i.e., operably linked to) certain control sequences such as promoter sequences, or sequences which may be inducible and/or cell type-specific. Suitable promoters will be known to a person with ordinary skill in the art. The expression construct will further contain sites for transcription initiation, termination and, in the transcribed region, a ribosome binding site for translation. When the DNA sequence described above is in the VEE replicon described above, the protein can be expressed *in vivo*. The DNA sequence for the *B. anthracis* MAT-PA protein described above can be cloned into the multiple cloning site of a replicon such that transcription of the RNA from the replicon yields an infectious RNA containing the *B. anthracis* protein of interest. Use of helper RNA containing sequences necessary for encapsulation of the viral transcript will result in the production of viral particles containing replicon RNA which are able to infect a host and initiate a single round of replication resulting in the expression of the *B. anthracis* proteins. Such replicon constructs include those listed in Table 1.

**Table 1**

| Replicon Plasmid Name | expresses |
|---|---|
| p3014-TPA-PA | TPA signal, 5 amino acids of C terminal end of prokaryotic secretory signal, and the 83kDa mature protein. |
| p3014-PA | 29 amino acid prokaryotic secretory signal, and the 83kDa mature protein secreted by *B. anthracis* |
| p3014-MAT-PA p3014-PA63 | 83kDa mature protein 63kDa protein (minus 20kDa N-terminal end that is naturally cleaved off by furin proteases at host cell membrane surface) |
| | |
| p3014-sub-domains | subdomain #1: from nucleotide 1891 to 2391; subdomain #2: from nucleotide 2392 to 2658; |
| | subdomain #3: from nucleotide 2659 to 3351; |
| | subdomain #4: from nucleotide 3352 to 3669; |
| | subdomain #5: from nucleotide 3670 to 4098 (Subdomains are numbered according to Welkos et al., 1988, supra; determined on subdomains present in the crystal structure, Petosa, et al., 1997, Nature 385, 833-838). |

The VEE constructs containing anthrax proteins can be used as a DNA vaccine, or for the production of RNA molecules as described below.

In another embodiment, the present invention relates to RNA molecules resulting from the transcription of the construct described above. The RNA molecules can be prepared by *in vitro* transcription using methods known in the art and described in the Examples below. Alternatively, the RNA molecules can be produced by transcription of the constructs *in vivo*, and isolating the RNA. These and other methods for obtaining RNA transcripts of the constructs are known in the art. Please see Current Protocols in Molecular Biology. Frederick M. Ausubel et al. (eds.), John Wiley and Sons, Inc. The RNA molecules can be used, for example, as a nucleic acid vaccine, or to transfect cells along with RNA from helper plasmids, one of which expresses VEE glycoproteins and the other VEE capsid proteins, as described above, in order to obtain replicon particles.

Introduction of the construct into the host cell can be effected by calcium phosphate transfection, electroporation, infection, and other methods known in the art and described in standard laboratory manuals such as Current Protocols in Molecular Biology, Ausubel, F. M. et al. (Eds), Wiley & Sons, Inc.

In a further embodiment, the present invention relates to host cells stably transformed or transfected with the above-described recombinant DNA construct. The host cell can be lower eukaryotic (for example, yeast or insect) or higher eukaryotic (for example, all mammals, including but not limited to rat and human). Eukaryotic host cells may be used for expression of desired coding sequences when appropriate control sequences which are compatible with.the designated host are used. Among prokaryotic hosts, *E. coli* is most frequently used. Expression control sequences for prokaryotes include promoters, optionally containing operator portions, and ribosome binding sites. Transfer vectors compatible with prokaryotic hosts are commonly derived from, for example, pBR322, a plasmid containing operons conferring ampicillin and tetracycline resistance, and the various pUC vectors, which also contain sequences conferring antibiotic resistance markers. These markers may be used to obtain successful transformants by selection. Please see e.g., Maniatis, Fitsch and Sambrook, Molecular Cloning; A Laboratory Manual (1982) or DNA Cloning, Volumes I and II (D. N. Glover ed. 1985) for general cloning methods. The DNA sequence can be present in the vector operably linked to a sequence encoding an IgG molecule, an adjuvant, a carrier, or an agent for aid in purification of protein of the invention, such as glutathione S-transferase. The recombinant molecule can be suitable for transfecting eukaryotic cells, for example, mammalian cells and yeast cells in culture systems. *Saccharomyces cerevisiae, Saccharomyces carlsbergensis,* and *Pichia pastoris* are the most commonly used yeast hosts, and are convenient fungal hosts. Control sequences for yeast vectors are known in the art. Mammalian cell lines available as hosts for expression are known in the art and include many immortalized cell lines available from the American Type Culture Collection (ATCC), such as baby hamster kidney (BHK) cells, MRC-5 cells and vero cells, to name a few. Suitable promoters are also known in the art and include viral promoters such as that from SV40, Rous sarcoma virus (RSV), adenovirus (ADV), bovine papilloma virus (BPV), and cytomegalovirus (CMV). Mammalian cells may also require terminator sequences and poly A addition sequences; enhancer sequences which increase expression may also be included, and sequences which cause amplification of the gene may also be desirable. These sequences are known in the art. The transformed or transfected host cells can be used as a source of DNA sequences described above. When the recombinant molecule takes the form of an expression system, the transformed or transfected cells can be used as a source of the protein specified as amino acid sequence in , SEQ ID NO:7 (MAT-PA).

A recombinant or derived polypeptide is not necessarily translated from a designated nucleic acid sequence; it may be generated in any manner, including for example, chemical synthesis, or expression of a recombinant expression system. In addition the polypeptide can be fused to other proteins or polypeptides which increase its antigenicity, such as adjuvants for example.

The recombinant or fusion protein can be used as a vaccine for immunity against anthrax infection or as a diagnostic tool for detection of bacillus anthracis. The transformed host cells can be used to analyze the effectiveness of drugs and agents which inhibit anthrax or *B. anthracis* proteins, such as host proteins which may interact with *B. anthracis* protein of the present invention to inhibit its function. A method for testing the effectiveness of an anti-anthrax drug or agent can for example be the rat anthrax toxin assay (Ivins et al. 1984, Infec. Immun. 52, 454-458 and Ivins et al. 1986) or a skin test in rabbits for assaying antiserum against anthrax toxin (Belton and Henderson, 1956, Br. J. Exp. Path. 37, 156-160).

In another embodiment, the present invention relates to an anthrax vaccine comprising one or more replicon particles derived from one or more replicons encoding *B. anthracis* MAT-PA protein having the amino acid sequence of SEQ ID NO:7. Immunity against anthrax is provided by administering one or more replicon particles containing any combination of the *B*. *anthracis* proteins to a subject such that a protective immune reaction is generated.

Vaccine formulations of the present invention comprise an immunogenic amount of a replicon particle, resulting from the replicon construct described above, in combination with a pharmaceutically acceptable carrier. An "immunogenic amount" is an amount of the replicon particles sufficient to evoke an immune response in the subject to which the vaccine is administered. An amount of from about 10² to 10⁷ per dose is suitable, more or less can be used depending upon the age and species of the subject being treated. Exemplary pharmaceutically acceptable carriers include, but are riot limited to, sterile pyrogen-free water and sterile pyrogen-free physiological saline solution.

Administration of the replicon particles disclosed herein may be carried out by any suitable means, including both parenteral injection (such as intraperitoneal, subcutaneous, or intramuscular injection), by in ovo injection in birds, orally and by topical application of the virus (typically carried in the pharmaceutical formulation) to an airway surface. Topical application of the virus to an airway surface can be carried out by intranasal administration (e.g. by use of dropper, swab, or inhaler which deposits a pharmaceutical formulation intranasally). Topical application of the virus to an airway surface can also be carried out by inhalation administration, such as by creating respirable particles of a pharmaceutical formulation (including both solid particles and liquid particles) containing the replicon as an aerosol suspension, and then causing the subject to inhale the respirable particles. Methods and apparatus for administering respirable particles of pharmaceutical formulations are well known, and any conventional technique can be employed. An "immunogenic amount" is an amount of the replicon particles sufficient to evoke an immune response in the subject to which the vaccine is administered.

When the replicon RNA or DNA is used as a vaccine, the replicon RNA or DNA can be administered directly using techniques such as delivery on gold beads (gene gun), delivery by liposomes, or direct injection, among other methods known to people in the art. Any one or more constructs or replicating RNA described above can be use in any combination effective to elicit an immunogenic response in a subject. Generally, the nucleic acid vaccine administered may be in an amount of about 1-5 ug of nucleic acid per dose and will depend on the subject to be treated, capacity of the subject's immune system to develop the desired immune response, and the degree of protection desired. Precise amounts of the vaccine to be administered may depend on the judgement of the practitioner and may be peculiar to each subject and antigen.

The vaccine may be given in a single dose schedule, or preferably a multiple dose schedule in which a primary course of vaccination may be with 1-10 separate doses, followed by other doses given at subsequent time intervals required to maintain and or reinforce the immune response, for example, at 1-4 months for a second dose, and if needed, a subsequent dose(s) after several months. Examples of suitable immunization schedules include: (i) 0, 1 months and 6 months, (ii) 0, 7 days and 1 month, (iii) 0 and 1 month, (iv) 0 and 6 months, or other schedules sufficient to elicit the desired immune responses expected to confer protective immunity, or reduce disease symptoms, or reduce severity of disease.

The following MATERIALS AND METHODS were used in the examples that follow.

**Plasmids.** Construction of the VEE replicon, capsid (C-) helper, and glycoprotein (GP-) helper plasmids was previously described (Pushko, 1997, supra). The PA gene from nucleotide 1804 to 4098 (SEQ ID NO:1) (Welkos et al., 1988, supra) was cloned into the VEE replicon plasmid as a BamHI/BamHI fragment utilizing a shuttle vector. TPA-PA (SEQ ID NO:3), MAT-PA (SEQ ID NO:2), and PA63 (SEQ ID NO:4) were PCR amplified using forward and reverse primers which also contained the ClaI recognition site. The PCR products were agarose gel purified and ligated into the T/A vector pCR2.1 (Invitrogen, Inc.). The PA genes, after digestion of pCR2.1-PA's with ClaI, were ligated into the VEE replicon plasmid. The Lassa nucleocapsid replicon (Lassa N-replicon) was constructed as previously described (Pushko, 1997, supra) and used as a negative control replicon.

**Production of VRP.** Plasmid templates for the PA-replicons, C-helper, GP-helper, and the Lassa N-replicon were linearized by digestion with NotI at a unique site downstream from the coding sequences, and capped run-off transcripts were prepared *in vitro* using T7 RNA polymerase. Packaging of the replicons into VEE replicon particles (VRPs) was accomplished by electroporating the replicon RNA and the two helper RNAs into BHK cells. VRPs were harvested between 20 and 27 hours after transfection and purified from cell culture supernatants by ultracentrifugation through a discontinuous sucrose gradient (20%). After reconstituting the pelleted VRP in 1/50 volume phosphate buffered saline, the VRPs were stored at - 70°C.

**Analysis of expression products and titration of VRP.** Subconfluent monolayers were infected with PA-VRP's (either TPA-PA, PA, MAT-PA, or PA63) or Lassa N-VRP (m.o.i. = 2) or cell suspensions were electroporated with replicon RNA. Cells were harvested at approximately 20-24 hours and expressed proteins were separated by SDS-PAGE. Visualization of PA proteins was accomplished using a chemiluminescence western blot assay and antibodies specific for each protein. Titration of VRPs was accomplished by infecting subconfluent monolayers with increasing dilutions of purified VRP. Antigen positive cells were visualized in an indirect immunofluorescence assay using a monoclonal antibody specific for each protein, or in a direct immunofluorescence assay using an FITC-conjugated monkey anti-Lassa serum.

**Immunization of mice.** Mice were inoculated 1 to 4 times at 28 day intervals with 10⁵ to 10⁷ infectious units (iu) of either PA-VRP or Lassa N-VRP (negative control). Positive control mice for the anthrax study were inoculated subcutaneously with 0.2 ml of anthrax vaccine at 28 day intervals. Serum for ELISA was obtained 2 days before each inoculation and 3 days before challenge.

### Enzyme-linked immunosorbent assay (ELISA).

Microtiter plates were coated with protective antigen (gift from Joe Farchaus, Bacteriology Division) (1 ug/ml) in PBS and allowed to absorb overnight at 4°C. Four fold serum dilutions in blocking buffer were applied to the plates and incubated at 37°C for 1 hour. After washing, an anti-mouse secondary antibody (HRP conjugated) was added to the plate and incubated for an additional hour at 37°C. After washing, bound antibody was detected colormetrically using ABTS as a substrate.

### Challenge of mice.

Anthrax challenge: C57BL/6 or A/J mice were challenged subcutaneously with 12-32 LD₅₀ *B. anthracis* (Sterne) 31 days after the last inoculation.

### Example 1

### Packaging and expression of PA-replicon

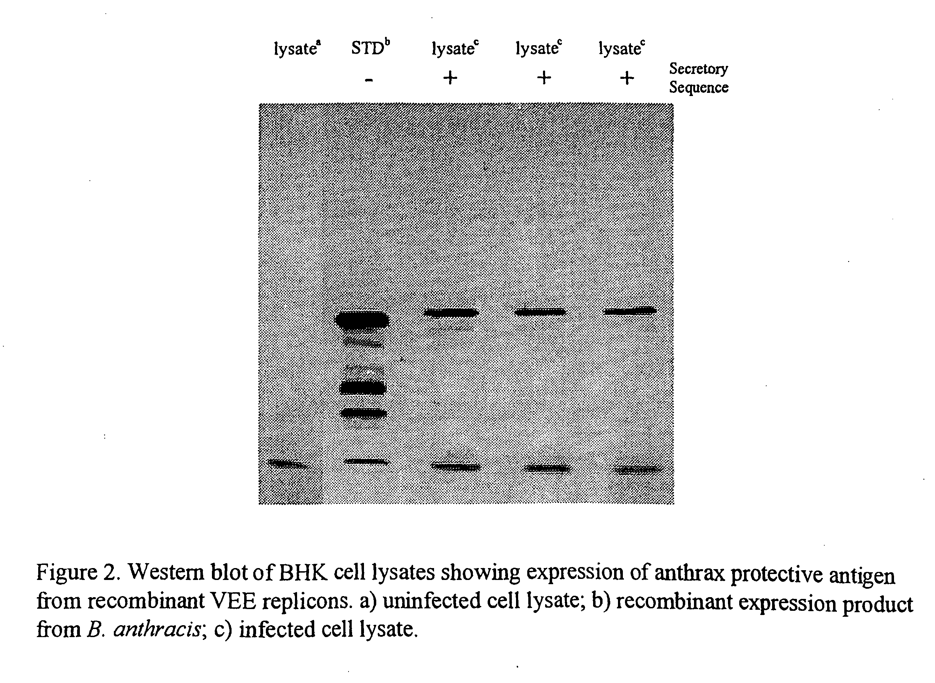

The PA-replicon was efficiently packaged into VRPs using the double helper system. Stock solutions contained about 10⁸ infectious units of purified PA-VRP per milliliter. No replication competent VEE virus was detected in any of the preparations. A western blot of cell lysates, generated from BHK cells infected with PA-VRP, demonstrates a product of approximately 85 kDa (Figure 2). Because the PA-VRP expression product contains a 29 amino acid prokaryotic secretory signal, which is cleaved from the standard, the PA product from the BHK cells appears larger than the PA standard.

### Example 2

### Protection against challenge with B. anthracis

The results of the animal studies demonstrated that PA from *B. anthracis* could be used to immunize and protect mice from a lethal challenge of *B. anthracis* (Sterne strain). Although the Sterne strain is attenuated in some animals, it is virulent in mice. Controls in the experiment included mice vaccinated with the current human vaccine (anthrax vaccine absorbed, AVA) which protected all mice, and mice immunized with the Lassa N-replicon which expressed an antigen irrelevant to anthrax protection which failed to protect the mice. Figure 3A shows ELISA titers and survival for C57B1/6 mice inoculated with two to four doses of PA-VRP. Seventy percent of the C57B1/6 mice which received three inoculations of the PA-VRP were protected whereas 90% of the C57B1/6 mice which received four inoculations were protected from an otherwise lethal challenge of *B. anthracis* (Sterne strain). The geometric mean of the serum ELISA titers to PA of C57B1/6 mice given four doses of the PA-replicon was 150500, as compared to 264 for mice that were inoculated with the negative control replicon. Figure 3B shows ELISA titers and survival for A/J mice inoculated with two to four doses of PA-VRP. Three inoculations of the PA-VRP protected 70% of the A/J mice, whereas four inoculations protected 90% of the A/J mice from an otherwise lethal challenge of the *B. anthracis* (Sterne strain). The geometric mean of the serum ELISA titers against PA in A/J mice given four doses of the PA-replicon was 223336 as compared to 100 for mice which were inoculated with the negative control replicon. As expected, none of the mice (from either strain) which were inoculated with the negative control replicon survived challenge with *B. anthracis* (Sterne strain).

### Discussion/Conclusion

Since VEE virus replicates in the cytoplasm of eukaryotic cells, the VEE replicon vaccine vector is a useful tool for the expression of prokaryotic genes in eukaryotic cells. Cytoplasmic expression of genes alleviates the difficulties imposed by splicing and nuclear transport of mRNA. We used the VEE replicon as a way to express the prokaryotic PA gene in eukaryotic cells and to develop a new vaccine candidate against anthrax.

The human AVA contains mostly PA protein and is presumed to protect humans by eliciting an antibody response that can neutralize the PA portion of anthrax toxin produced by invading *B. anthracis.* After neutralization of the toxin, the immune system destroys the invading bacteria. In order to model the human disease, we chose to use mice and the Sterne strain of *B. anthracis.* The Sterne strain produces anthrax toxin (encoded on the pX01 plasmid) but lacks a capsule (encoded on the pX02 plasmid). Mice exposed to large quantities of Sterne (ranging from 10³ to 10⁸ spores for the different mouse strains) are not able to overcome the effects of the toxin produced by the bacteria so they succumb to infection. We hypothesized that by immunizing the mice with PA, thereby inducing neutralizing antibodies to the PA portion of the toxin produced by a challenge inoculum, that the mice would be protected.

A VEE replicon was constructed which expressed the PA gene from *B. anthracis* and was evaluated for *in vitro* expression, *in vivo* immunogenicity, and protective efficacy. C57B1/6 and A/J mice demonstrated increasing antibody responses to PA when inoculated with increasing doses of PA-VRP. The two mouse strains inoculated with up to four doses of PA-VRP displayed increased protection against a lethal challenge with *B. anthracis* (Sterne strain). The use of mice and the Sterne strain of *B. anthracis* useful as a model system for studying the immunogenicity and efficacy of replicon based anthrax vaccines.

### TPA-PA amino acid sequence SEQ ID NO:5

1 MDAMKRGLCCVLLLCGAVFVSASEVIQAEVKQENRLLNESESS
44 SQGLLGYYFSDLNFQAPMVVTSSTTGDLSIPSSELENIPSENQYFQSAIWSGFIKVKK
102 SDEYTFATSADNHVTMWVDDQEVINKASNSNKIRLEKGRLYQIKIQYQRENPTEKGLD
160 FKLYWTDSQNKKEVISSDNLQLPELKQKSSNSRKKRSTSAGPTVPDRDNDGIPDSLEV
218 EGYTVDVKNKRTFLSPWISNIHEKKGLTKYKSSPEKWSTASDPYSDFEKVTGRIDKNV
276 SPEARHPLVAAYPIVHVDMENIILSKNEDQSTQNTDSETRTISKNTSTSRTHTSEVHG
334 NAEVHASFFDIGGSVSAGFSNSNSSTVAIDHSLSLAGERTWAETMGLNTADTARLNAN
392 IRYVNTGTAPIYNVLPTTSLVLGKNQTLATIKAKENQLSQILAPNNYYPSKNLAPIAL
450 NAQDDFSSTPITMNYNQFLELEKTKQLRLDTDQVYGNIATYNFENGRVRVDTGSNWSE
508 VLPQIQETTARIIFNGKDLNLVERRIAAVNPSDPLETTKPDMTLKEALKIAFGFNEPN
566 GNLQYQGKDITEFDFNFDQQTSQNIKNQLAELNATNIYTVLDKIKLNAKMNILIRDKR
624 FHYDRNNIAVGADESVVKEAHREVINSSTEGLLLNIDKDIRKILSGYIVEIEDTEGLK
682 EVINDRYDMLNISSLRQDGKTFIDFKKYNDKLPLYISNPNYKVNVYAVTKENTIINPS
706 ENGDTSTNGIKKILIFSKKGYEIG

### TPA-PA gene sequence SEQ ID NO:3

1 atgg atgcaatgaa gagagggctc tgctgtgtgc tgctgctgtg tggagcagtc
55 ttcgtttcgg ctagcgaggt gattcaggca gaagttaaac aggagaaccg gttattaaat
115 gaatcagaat caagttccca ggggttacta ggatactatt ttagtgattt gaattttcaa
175 gcacccatgg tggttacctc ttctactaca ggggatttat ctattcctag ttctgagtta
235 gaaaatattc catcggaaaa ccaatatttt caatctgcta tttggtcagg atttatcaaa
295 gttaagaaga gtgatgaata tacatttgct acttccgctg ataatcatgt aacaatgtgg
355 gtagatgacc aagaagtgat taataaagct tctaattcta acaaaatcag attagaaaaa
415 ggaagattat atcaaataaa aattcaatat caacgagaaa atcctactga aaaaggattg
475 gatttcaagt tgtactggac cgattctcaa aataaaaaag aagtgatttc tagtgataac
535 ttacaattgc cagaattaaa acaaaaatct tcgaactcaa gaaaaaagcg aagtacaagt
595 gctggaccta cggttccaga ccgtgacaat gatggaatcc ctgattcatt agaggtagaa
655 ggatatacgg ttgatgtcaa aaataaaaga acttttcttt caccatggat ttctaatatt
715 catgaaaaga aaggattaac caaatataaa tcatctcctg aaaaatggag cacggcttct
775 gatccgtaca gtgatttcga aaaggttaca ggacggattg ataagaatgt atcaccagag
835 gcaagacacc cccttgtggc agcttatccg attgtacatg tagatatgga gaatattatt
895 ctctcaaaaa atgaggatca atccacacag aatactgata gtgaaacgag aacaataagt
955 aaaaatactt ctacaagtag gacacatact agtgaagtac atggaaatgc agaagtgcat
1015 gcgtcgttct ttgatattgg tgggagtgta tctgcaggat ttagtaattc gaattcaagt
1075 acggtcgcaa ttgatcattc actatctcta gcaggggaaa gaacttgggc tgaaacaatg
1135 ggtttaaata ccgctgatac agcaagatta aatgccaata ttagatatgt aaatactggg
1195 acggctccaa tctacaacgt gttaccaacg acttcgttag tgttaggaaa aaatcaaaca
1255 ctcgcgacaa ttaaagctaa ggaaaaccaa ttaagtcaaa tacttgcacc taataattat
1315 tatccttcta aaaacttggc gccaatcgca ttaaatgcac aagacgattt cagttctact
1375 ccaattacaa tgaattacaa tcaatttctt gagttagaaa aaacgaaaca attaagatta
1435 gatacggatc aagtatatgg gaatatagca acatacaatt ttgaaaatgg aagagtgagg
1495 gtggatacag gctcgaactg gagtgaagtg ttaccgcaaa ttcaagaaac aactgcacgt
1555 atcattttta atggaaaaga tttaaatctg gtagaaaggc ggatagcggc ggttaatcct
1615 agtgatccat tagaaacgac taaaccggat atgacattaa aagaagccct taaaatagca
1675 tttggattta acgaaccgaa tggaaactta caatatcaag ggaaagacat aaccgaattt
1735 gattttaatt tcgatcaaca aacatctcaa aatatcaaga atcagttagc ggaattaaac
1795 gcaactaaca tatatactgt attagataaa atcaaattaa atgcaaaaat gaatatttta
1855 ataagagata aacgttttca ttatgataga aataacatag cagttggggc ggatgagtca
1915 gtagttaagg aggctcatag agaagtaatt aattcgtcaa cagagggatt attgttaaat
1975 attgataagg atataagaaa aatattatca ggttatattg tagaaattga agatactgaa
2035 gggcttaaag aagttataaa tgacagatat gatatgttga atatttctag tttacggcaa
2095 gatggaaaaa catttataga ttttaaaaaa tataatgata aattaccgtt atatataagt
2155 aatcccaatt ataaggtaaa tgtatatgct gttactaaag aaaacactat tattaatcct
2215 agtgagaatg gggatactag taccaacggg atcaagaaaa ttttaatctt ttctaaaaaa
2275 ggctatgaga taggataa

### PA amino acid sequence SEQ ID NO:6

1 MKKRKVLIPLMALSTILVSSTGNLEVIQAEVKQENRLLNESESS
45 SQGLLGYYFSDLNFQAPMVVTSSTTGDLSIPSSELENIPSENQYFQSAIWSGFIKVKK
103 SDEYTFATSADNHVTMWVDDQEVINKASNSNKIRLEKGRLYQIKIQYQRENPTEKGLD
161 FKLYWTDSQNKKEVISSDNLQLPELKQKSSNSRKKRSTSAGPTVPDRDNDGIPDSLEV
219 EGYTVDVKNKRTFLSPWISNIHEKKGLTKYKSSPEKWSTASDPYSDFEKVTGRIDKNV
277 SPEARHPLVAAYPIVHVDMENIILSKNEDQSTQNTDSETRTISKNTSTSRTHTSEVHG
335 NAEVHASFFDIGGSVSAGFSNSNSSTVAIDHSLSLAGERTWAETMGLNTADTARLNAN
393 IRYVNTGTAPIYNVLPTTSLVLGKNQTLATIKAKENQLSQILAPNNYYPSKNLAPIAL
451 NAQDDFSSTPITMNYNQFLELEKTKQLRLDTDQVYGNIATYNFENGRVRVDTGSNWSE
509 VLPQIQETTARIIFNGKDLNLVERRIAAVNPSDPLETTKPDMTLKEALKIAFGFNEPN
567 GNLQYQGKDITEFDFNFDQQTSQNIKNQLAELNATNIYTVLDKIKLNAKMNILIRDKR
625 FHYDRNNIAVGADESVVKEAHREVINSSTEGLLLNIDKDIRKILSGYIVEIEDTEGLK
683 EVINDRYDMLNISSLRQDGKTFIDFKKYNDKLPLYISNPNYKVNVYAVTKENTIINPS
707 ENGDTSTNGIKKILIFSKKGYEIG

### PA gene sequence SEQ ID NO:1

1 atgaaaa aacgaaaagt gttaatacca ttaatggcat tgtctacgat attagtttca
58 agcacaggta atttagaggt gattcaggca gaagttaaac aggagaaccg gttattaaat
118 gaatcagaat caagttccca ggggttacta ggatactatt ttagtgattt gaattttcaa
178 gcacccatgg tggttacctc ttctactaca ggggatttat ctattcctag ttctgagtta
238 gaaaatattc catcggaaaa ccaatatttt caatctgcta tttggtcagg atttatcaaa
298 gttaagaaga gtgatgaata tacatttgct acttccgctg ataatcatgt aacaatgtgg
358 gtagatgacc aagaagtgat taataaagct tctaattcta acaaaatcag attagaaaaa
418 ggaagattat atcaaataaa aattcaatat caacgagaaa atcctactga aaaaggattg
478 gatttcaagt tgtactggac cgattctcaa aataaaaaag aagtgatttc tagtgataac
538 ttacaattgc cagaattaaa acaaaaatct tcgaactcaa gaaaaaagcg aagtacaagt
598 gctggaccta cggttccaga ccgtgacaat gatggaatcc ctgattcatt agaggtagaa
658 ggatatacgg ttgatgtcaa aaataaaaga acttttcttt caccatggat ttctaatatt
718 catgaaaaga aaggattaac caaatataaa tcatctcctg aaaaatggag cacggcttct
778 gatccgtaca gtgatttcga aaaggttaca ggacggattg ataagaatgt atcaccagag
838 gcaagacacc cccttgtggc agcttatccg attgtacatg tagatatgga gaatattatt
898 ctctcaaaaa atgaggatca atccacacag aatactgata gtgaaacgag aacaataagt
958 aaaaatactt ctacaagtag gacacatact agtgaagtac atggaaatgc agaagtgcat
1018 gcgtcgttct ttgatattgg tgggagtgta tctgcaggat ttagtaattc gaattcaagt
1078 acggtcgcaa ttgatcattc actatctcta gcaggggaaa gaacttgggc tgaaacaatg
1138 ggtttaaata ccgctgatac agcaagatta aatgccaata ttagatatgt aaatactggg
1198 acggctccaa tctacaacgt gttaccaacg acttcgttag tgttaggaaa aaatcaaaca
1258 ctcgcgacaa ttaaagctaa ggaaaaccaa ttaagtcaaa tacttgcacc taataattat
1318 tatccttcta aaaacttggc gccaatcgca ttaaatgcac aagacgattt cagttctact
1378 ccaattacaa tgaattacaa tcaatttctt gagttagaaa aaacgaaaca attaagatta
1438 gatacggatc aagtatatgg gaatatagca acatacaatt ttgaaaatgg aagagtgagg
1498 gtggatacag gctcgaactg gagtgaagtg ttaccgcaaa ttcaagaaac aactgcacgt
1558 atcattttta atggaaaaga tttaaatctg gtagaaaggc ggatagcggc ggttaatcct
1618 agtgatccat tagaaacgac taaaccggat atgacattaa aagaagccct taaaatagca
1678 tttggattta acgaaccgaa tggaaactta caatatcaag ggaaagacat aaccgaattt
1738 gattttaatt tcgatcaaca aacatctcaa aatatcaaga atcagttagc ggaattaaac
1798 gcaactaaca tatatactgt attagataaa atcaaattaa atgcaaaaat gaatatttta
1858 ataagagata aacgttttca ttatgataga aataacatag cagttggggc ggatgagtca
1918 gtagttaagg aggctcatag agaagtaatt aattcgtcaa cagagggatt attgttaaat
1978 attgataagg atataagaaa aatattatca ggttatattg tagaaattga agatactgaa
2038 gggcttaaag aagttataaa tgacagatat gatatgttga atatttctag tttacggcaa
2098 gatggaaaaa catttataga ttttaaaaaa tataatgata aattaccgtt atatataagt
2158 aatcccaatt ataaggtaaa tgtatatgct gttactaaag aaaacactat tattaatcct
2218 agtgagaatg gggatactag taccaacggg atcaagaaaa ttttaatctt ttctaaaaaa
2278 ggctatgaga taggataa

### MAT-PA amino acid sequence SEQ ID NO:7

1 MEVKQENRLLNESESS
17 SQGLLGYYFSDLNFQAPMVVTSSTTGDLSIPSSELENIPSENQYFQSAIWSGFIKVKK
75 SDEYTFATSADNHVTMWVDDQEVINKASNSNKIRLEKGRLYQIKIQYQRENPTEKGLD
133 FKLYWTDSQNKKEVISSDNLQLPELKQKSSNSRKKRSTSAGPTVPDRDNDGIPDSLEV
191 EGYTVDVKNKRTFLSPWISNIHEKKGLTKYKSSPEKWSTASDPYSDFEKVTGRIDKNV
249 SPEARHPLVAAYPIVHVDMENIILSKNEDQSTQNTDSETRTISKNTSTSRTHTSEVHG
307 NAEVHASFFDIGGSVSAGFSNSNSSTVAIDHSLSLAGERTWAETMGLNTADTARLNAN
365 IRYVNTGTAPIYNVLPTTSLVLGKNQTLATIKAKENQLSQILAPNNYYPSKNLAPIAL
423 NAQDDFSSTPITMNYNQFLELEKTKQLRLDTDQVYGNIATYNFENGRVRVDTGSNWSE
481 VLPQIQETTARIIFNGKDLNLVERRIAAVNPSDPLETTKPDMTLKEALKIAFGFNEPN
539 GNLQYQGKDITEFDFNFDQQTSQNIKNQLAELNATNIYTVLDKIKLNAKMNILIRDKR
597 FHYDRNNIAVGADESVVKEAHREVINSSTEGLLLNIDKDIRKILSGYIVEIEDTEGLK
655 EVINDRYDMLNISSLRQDGKTFIDFKKYNDKLPLYISNPNYKVNVYAVTKENTIINPS
679 ENGDTSTNGIKKILIFSKKGYEIG

### MAT-PA gene sequence SEQ ID NO:2

1                                atg gaagttaaac aggagaaccg gttattaaat
  34 gaatcagaat caagttccca ggggttacta ggatactatt ttagtgattt gaattttcaa
  94 gcacccatgg tggttacctc ttctactaca ggggatttat ctattcctag ttctgagtta
 154 gaaaatattc catcggaaaa ccaatatttt caatctgcta tttggtcagg atttatcaaa
 214 gttaagaaga gtgatgaata tacatttgct acttccgctg ataatcatgt aacaatgtgg
 274 gtagatgacc aagaagtgat taataaagct tctaattcta acaaaatcag attagaaaaa
 334 ggaagattat atcaaataaa aattcaatat caacgagaaa atcctactga aaaaggattg
 394 gatttcaagt tgtactggac cgattctcaa aataaaaaag aagtgatttc tagtgataac
 454 ttacaattgc cagaattaaa acaaaaatct tcgaactcaa gaaaaaagcg aagtacaagt
 414 gctggaccta cggttccaga ccgtgacaat gatggaatcc ctgattcatt agaggtagaa
 474 ggatatacgg ttgatgtcaa aaataaaaga acttttcttt caccatggat ttctaatatt
 534 catgaaaaga aaggattaac caaatataaa tcatctcctg aaaaatggag cacggcttct
 594 gatccgtaca gtgatttcga aaaggttaca ggacggattg ataagaatgt atcaccagag
 654 gcaagacacc cccttgtggc agcttatccg attgtacatg tagatatgga gaatattatt
 714 ctctcaaaaa atgaggatca atccacacag aatactgata gtgaaacgag aacaataagt
 774 aaaaatactt ctacaagtag gacacatact agtgaagtac atggaaatgc agaagtgcat
 834 gcgtcgttct ttgatattgg tgggagtgta tctgcaggat ttagtaattc gaattcaagt
 894 acggtcgcaa ttgatcattc actatctcta gcaggggaaa gaacttgggc tgaaacaatg
 954 ggtttaaata ccgctgatac agcaagatta aatgccaata ttagatatgt aaatactggg
1014 acggctccaa tctacaacgt gttaccaacg acttcgttag tgttaggaaa aaatcaaaca
1074 ctcgcgacaa ttaaagctaa ggaaaaccaa ttaagtcaaa tacttgcacc taataattat
1134 tatccttcta aaaacttggc gccaatcgca ttaaatgcac aagacgattt cagttctact
1194 ccaattacaa tgaattacaa tcaatttctt gagttagaaa aaacgaaaca attaagatta
1254 gatacggatc aagtatatgg gaatatagca acatacaatt ttgaaaatgg aagagtgagg
1314 gtggatacag gctcgaactg gagtgaagtg ttaccgcaaa ttcaagaaac aactgcacgt
1374 atcattttta atggaaaaga tttaaatctg gtagaaaggc ggatagcggc ggttaatcct
1434 agtgatccat tagaaacgac taaaccggat atgacattaa aagaagccct taaaatagca
1494 tttggattta acgaaccgaa tggaaactta caatatcaag ggaaagacat aaccgaattt
1554 gattttaatt tcgatcaaca aacatctcaa aatatcaaga atcagttagc ggaattaaac
1614 gcaactaaca tatatactgt attagataaa atcaaattaa atgcaaaaat gaatatttta
1674 ataagagata aacgttttca ttatgataga aataacatag cagttggggc ggatgagtca
1734 gtagttaagg aggctcatag agaagtaatt aattcgtcaa cagagggatt attgttaaat
1794 attgataagg atataagaaa aatattatca ggttatattg tagaaattga agatactgaa
1854 gggcttaaag aagttataaa tgacagatat gatatgttga atatttctag tttacggcaa
1914 gatggaaaaa catttataga ttttaaaaaa tataatgata aattaccgtt atatataagt
1974 aatcccaatt ataaggtaaa tgtatatgct gttactaaag aaaacactat tattaatcct
2034 agtgagaatg gggatactag taccaacggg atcaagaaaa ttttaatctt ttctaaaaaa
2094 ggctatgaga taggataa

### PA63 amino acid sequence SEQ ID NO:8

1 MSTSAGPTVPDRDNDGIPDSLEV
24 EGYTVDVKNKRTFLSPWISNIHEKKGLTKYKSSPEKWSTASDPYSDFEKVTGRIDKNV
82 SPEARHPLVAAYPIVHVDMENIILSKNEDQSTQNTDSETRTISKNTSTSRTHTSEVHG
140 NAEVHASFFDIGGSVSAGFSNSNSSTVAIDHSLSLAGERTWAETMGLNTADTARLNAN
198 IRYVNTGTAPIYNVLPTTSLVLGKNQTLATIKAKENQLSQILAPNNYYPSKNLAPIAL
256 NAQDDFSSTPITMNYNQFLELEKTKQLRLDTDQVYGNIATYNFENGRVRVDTGSNWSE
314 VLPQIQETTARIIFNGKDLNLVERRIAAVNPSDPLETTKPDMTLKEALKIAFGFNEPN
372 GNLQYQGKDITEFDFNFDQQTSQNIKNQLAELNATNIYTVLDKIKLNAKMNILIRDKR
430 FHYDRNNIAVGADESVVKEAHREVINSSTEGLLLNIDKDIRKILSGYIVEIEDTEGLK
488 EVINDRYDMLNISSLRQDGKTFIDFKKYNDKLPLYISNPNYKVNVYAVTKENTIINPS
546 ENGDTSTNGIKKILIFSKKGYEIG

### PA63 gene sequence SEQ ID NO:4

1                                                    at gagtacaagt
  13 gctggaccta cggttccaga ccgtgacaat gatggaatcc ctgattcatt agaggtagaa
  73 ggatatacgg ttgatgtcaa aaataaaaga acttttcttt caccatggat ttctaatatt
 133 catgaaaaga aaggattaac caaatataaa tcatctcctg aaaaatggag cacggcttct
 193 gatccgtaca gtgatttcga aaaggttaca ggacggattg ataagaatgt atcaccagag
 253 gcaagacacc cccttgtggc agcttatccg attgtacatg tagatatgga gaatattatt
 313 ctctcaaaaa atgaggatca atccacacag aatactgata gtgaaacgag aacaataagt
 373 aaaaatactt ctacaagtag gacacatact agtgaagtac atggaaatgc agaagtgcat
 433 gcgtcgttct ttgatattgg tgggagtgta tctgcaggat ttagtaattc gaattcaagt
 493 acggtcgcaa ttgatcattc actatctcta gcaggggaaa gaacttgggc tgaaacaatg
 553 ggtttaaata ccgctgatac agcaagatta aatgccaata ttagatatgt aaatactggg
 613 acggctccaa tctacaacgt gttaccaacg acttcgttag tgttaggaaa aaatcaaaca
 673 ctcgcgacaa ttaaagctaa ggaaaaccaa ttaagtcaaa tacttgcacc taataattat
 733 tatccttcta aaaacttggc gccaatcgca ttaaatgcac aagacgattt cagttctact
 793 ccaattacaa tgaattacaa tcaatttctt gagttagaaa aaacgaaaca attaagatta
 853 gatacggatc aagtatatgg gaatatagca acatacaatt ttgaaaatgg aagagtgagg
 913 gtggatacag gctcgaactg gagtgaagtg ttaccgcaaa ttcaagaaac aactgcacgt
 973 atcattttta atggaaaaga tttaaatctg gtagaaaggc ggatagcggc ggttaatcct
1033 agtgatccat tagaaacgac taaaccggat atgacattaa aagaagccct taaaatagca
1093 tttggattta acgaaccgaa tggaaactta caatatcaag ggaaagacat aaccgaattt
1153 gattttaatt tcgatcaaca aacatctcaa aatatcaaga atcagttagc ggaattaaac
1213 gcaactaaca tatatactgt attagataaa atcaaattaa atgcaaaaat gaatatttta
1273 ataagagata aacgttttca ttatgataga aataacatag cagttggggc ggatgagtca
1333 gtagttaagg aggctcatag agaagtaatt aattcgtcaa cagagggatt attgttaaat
1393 attgataagg atataagaaa aatattatca ggttatattg tagaaattga agatactgaa
1453 gggcttaaag aagttataaa tgacagatat gatatgttga atatttctag tttacggcaa
1513 gatggaaaaa catttataga ttttaaaaaa tataatgata aattaccgtt atatataagt
1573 aatcccaatt ataaggtaaa tgtatatgct gttactaaag aaaacactat tattaatcct
1633 agtgagaatg gggatactag taccaacggg atcaagaaaa ttttaatctt ttctaaaaaa
1693 ggctatgaga taggataa

## Claims

1. A recombinant DNA construct comprising:
(i) a VEE virus replicon vector, and
(ii) at least one nucleic acid fragment encoding *B. anthracis* MAT-PA protein having the amino acid sequence of SEQ ID NO:7.

2. A recombinant DNA construct according to claim 1 wherein said vector is an expression vector.

3. Self replicating RNA produced from the construct of claim 1.

4. Infectious alphavirus particles produced from packaging the self replicating RNA of claim 3.

5. A pharmaceutical composition comprising infectious alphavirus particles according to claim 4 in an effective immunogenic amount in a pharmaceutically acceptable carrier and/or adjuvant.

6. A host cell transformed with a recombinant DNA construct according to claims 1 or 2.

7. A host cell according to claim 6 wherein said host cell is eukaryotic.

8. A method for producing *B. anthracis* MAT-PA protein having the amino acid sequence of SEQ ID NO: 7, comprising culturing the cells according to claim 7 under conditions such that said DNA fragment is expressed and said protein is produced.

9. A vaccine for anthrax comprising viral particles containing one or more replicon RNA encoding *B. anthracis* MAT-PA protein having the amino acid sequence of SEQ ID NO: 7.

10. A pharmaceutical composition comprising the self replication RNA of claim 3 in an effective immunogenic amount in a pharmaceutically acceptable carrier and/or adjuvant.

11. A pharmaceutical composition comprising the recombinant DNA construct of claim 1 in a pharmaceutically acceptable amount, in a pharmaceutically acceptable carrier and/or adjuvant.

## Patentansprüche

1. Ein rekombinantes DNA-Konstrukt, umfassend:
(i) einen VEE Virus Replikonvektor, und
(ii) mindestens ein Nukleinsäurefragment, kodierend für *B. anthracis* MAT-PA-Protein, welches die Aminosäuresequenz von SEQ ID NO: 7 aufweist.

2. Ein rekombinantes DNA-Konstrukt nach Anspruch 1, **dadurch gekennzeichnet, dass** der Vektor ein Expressionsvektor ist.

3. Sich selbst replizierende RNA, hergestellt von dem Konstrukt nach Anspruch 1.

4. Infektiöse Alphaviruspartikel, hergestellt durch Verpacken der sich selbst replizierenden RNA nach Anspruch 3.

5. Eine pharmazeutische Zusammensetzung, umfassend infektiöse Alphaviruspartikel nach Anspruch 4 in einer effektiven immunogenen Menge in einem pharmazeutisch akzeptablen Träger und/oder Adjuvans.

6. Eine Wirtszelle transformiert mit einem rekombinanten DNA-Konstrukt nach den Ansprüchen 1 oder 2.

7. Ein Wirtszelle nach Anspruch 6, **dadurch gekennzeichnet, dass** die Wirtszelle eukaryotisch ist.

8. Ein Verfahren zur Herstellung von *B. anthracis* MAT-PA-Protein, welches die Aminosäuresequenz von SEQ ID NO: 7 aufweist, Schritte umfassend, bei denen man die Zellen nach Anspruch 7 unter solchen Bedingungen kultiviert, dass das DNA-Fragment exprimiert wird und das Protein hergestellt wird.

9. Ein Impfstoff gegen Anthrax, umfassend virale Partikel, welche ein oder mehrere Replikons RNA enthalten, die für *B. anthracis* MAT-PA-Protein kodiert, welches die Aminosäuresequenz von SEQ ID NO: 7 aufweist.

10. Eine pharmazeutische Zusammensetzung, umfassend die sich selbst replizierende RNA nach Anspruch 3 in einer effektiven immunogenen Menge in einem pharmazeutisch akzeptablen Träger und/oder Adjuvans.

11. Eine pharmazeutische Zusammensetzung, umfassend das rekombinante DNA-Konstrukt nach Anspruch 1 in einer pharmazeutisch akzeptablen Menge, in einem pharmazeutisch akzeptablen Träger und/oder Adjuvans.

## Revendications

1. Construction d'ADN recombiné, comprenant :
i) un vecteur qui est un réplicon du virus VEE,
ii) et au moins un fragment d'acide nucléique codant la protéine MAT-PA de *B. anthracis* qui présente la séquence d'acides aminés donnée en tant que Séquence N° 7.

2. Construction d'ADN recombiné, conforme à la revendication 1, dans laquelle ledit vecteur est un vecteur d'expression.

3. ARN auto-répliquant, produit à partir d'une construction conforme à la revendication 1.

4. Particules infectieuses d'alphavirus, produites par encapsidation d'un ARN auto-répliquant conforme à la revendication 3.

5. Composition pharmaceutique comprenant des particules infectieuses d'alphavirus, conformes à la revendication 4, en une quantité à effet immunogène, dans un véhicule et/ou un adjuvant pharmacologiquement admissible(s).

6. Cellule hôte transformée avec une construction d'ADN recombiné conforme à la revendication 1 ou 2.

7. Cellule hôte conforme à la revendication 6, laquelle cellule hôte est une cellule eucaryote.

8. Procédé de production de la protéine MAT-PA de *B. anthracis,* présentant la séquence d'acides aminés donnée en tant que Séquence N° 7, lequel procédé comporte le fait de cultiver des cellules conformes à la revendication 7 dans des conditions telles que ledit fragment d'ADN soit exprimé et que ladite protéine soit produite.

9. Vaccin contre le charbon, comprenant des particules virales qui contiennent un ou plusieurs ARN de réplicon codant la protéine MAT-PA de *B. anthracis* qui présente la séquence d'acides aminés donnée en tant que Séquence N° 7.

10. Composition pharmaceutique comprenant un ARN auto-répliquant, conforme à la revendication 3, en une quantité à effet immunogène, dans un véhicule et/ou un adjuvant pharmacologiquement admissible(s).

11. Composition pharmaceutique comprenant une construction d'ADN recombiné, conforme à la revendication 1, en une quantité à effet immunogène, dans un véhicule et/ou un adjuvant pharmacologiquement admissible(s).
